# EUROPEAN PATENT APPLICATION

(11) **EP 4 847 042 A1**
(43) Date of publication of application: **02.12.2026**
(21) Application number: 25224381.1
(22) Date of filing: 17.12.2025
(51) Int. Cl.: A61K 40/11, A61K 40/31, A61K 40/42, A61K 40/50

(54) **MINIMALLY MANIPULATED UNIVERSAL CAR-T CELL LACKING MHC CLASS I MOLECULES**

(30) Priority: 27.05.2025 TR 2025006877
(71) Applicant: Celltek Saglik Ve Danismanlik Hizmetleri Limited Sirketi, Istanbul (TR); Acibadem Mehmet Ali Aydinlar Üniversitesi, Atasehir/Istanbul (TR); Acibadem Labmed Saglik Hizmetleri Anonim Sirketi, 34752 Atasehir/Istanbul (TR)
(72) Inventor: OVALI, Ercüment, Istanbul (TR); HEM NL O LU, Cansu, Istanbul (TR); ÖZDEM R, Sevda Ye im, Istanbul (TR); GÖK, Faysal, Ankara (TR)
(74) Representative: Yamankaradeniz, Kemal

(57) **Abstract**

The invention relates to a method for obtaining minimally manipulated universal CAR-T cell (UN CAR-T CELL) lacking MHC class I molecules, which can be applied across a wide range of indications including cell therapy platform, hematological malignancies, solid tumors, other oncological diseases and autoimmune disorders. In particular, the invention concerns the generation of hundreds of distinct universal CAR-T cell types targeting different antigens by introducing any desired CAR construct into minimally manipulated universal T cells obtained from a donor lacking MHC class I molecules (MHC-NULL) for use in CAR-T cell applications.

## Description

### Technical Field

The invention relates to a method for obtaining a minimally Manipulated Universal CAR-T cell (UN CAR-T CELL) that lacks MHC class I molecules, which is applicable to a broad range of indications, including cell therapy platform, hematologic malignancies, solid tumors, other oncological diseases, and autoimmune disorders.

The invention specifically relates to the generation of hundreds of distinct universal CAR-T cells, each targeting different antigens by introducing any desired CAR construct into minimally manipulated universal T cells derived from a donor lacking MHC class I molecule -specifically, an individual diagnosed with ***Bare Lymphocyte Syndrome Type 1*,** for use in CAR-T cell therapies.

### State of Art

CAR-T cell therapy is a groundbreaking form of immunotherapy that has revolutionized cancer treatment. This approach involves the genetic modification of a patient's own immune cells-specifically, T cells-so they can recognize and eliminate cancer cells. The term "CAR" stands for Chimeric Antigen Receptor, a receptor expressed on the surface of T cells that enables them to identify and bind to cancer-specific targets.

Currently, CAR-T Cells are manufactured on a patient specific-bases using either the patient's own cells (autologous) or in a fewer cases cells from an allogeneic donor, if avaliable. When generated from the patient's own cells (Autologous CAR-T Cell), the following challenges arise;
a. T lymphocytes required for CAR-T cell production may be insufficient in number due to prior treatments or may exhibit functional impairments, making the manufacturing process inefficient.
b. Even in cases where adequate T cells can be obtained from the patient, three major issues still occur:
   - Due to the patient's underlying disease and/or prior exposure to chemotherapy or radiotherapy, the initial quality of the T cells is often poor which negatively impacts the quality of the final CAR-T product.
   - Upon completion of manufacturing, these suboptimal T cells are compelled to undergo extensive proliferation to achieve the required cell dose, a process that often results in T cell exhaustion and/or functional impairment.
   - The production of CAR-T cells obtained from a patient's own T lymphocytes typically takes an average of 8 to 15 days, and when quality control testing is included, the total process can exceed 20 days before the product can be released.This time frame can be clinically critical and in some cases, patients may die before the treatment is ready for administration.

Allogeneic (using cells from a bone marrow transplant donor) CAR-T Cell production presents two main challenges;
∘ Similar to autologous production, this approach also results in a significant loss of time, with the delivery of the product to the patient taking more than 20 days.
∘ Allogeneic T cells, when introduced into a foreign host, can trigger severe and potentially life-threatening immune reactions, a condition known as Graft-versus-Host Disease (GVHD).

One of the common challenges associated with the aforementioned methods is that CAR-T cells must be manufactured individually for each patient, which leads to significantly high costs. To overcome these issues, the Classical Universal CAR-T Cell (KUCT) platform was developed. However, unlike autologous or allogeneic CAR-T cells, there is currently no clinically approved, minimally manipulated universal CAR-T cell product. The KUCT products developed to date present the following problems:
1. In order to prevent the development of GVHD in these products, T cell receptors (TCR) are knocked out using CRISPR-Cas9 or similar genetic modification technologies.
2. In order to prevent the recipient's immune system from eliminating the KUCT cells, major histocompatibility complex (MHC) antigens are also deleted using CRISPR-Cas9 or equivalent gene-editing tools. However, these modifications render the KUCT cells vulnerable to attacks by the patient's natural killer (NK) cells. To address this, KIR (Killer-cell Immunoglobulin-like Receptor) molecules that inhibit NK cells are introduced on the cell surface via lentiviral vectors or other genetic modification techniques.

In addition to these three major genetic alterations, further modifications are applied during the insertion of the CAR molecule-typically using lentiviral or other vector-based systems. As a result, KUCT cells become excessively genetically manipulated, artificial in nature, of questionable safety, and carry a heightened risk of oncogenic transformation. Although the use of a single source or donor to manufacture products for multiple patients may appear cost-effective from a production standpoint, the extensive genetic modifications required significantly increase overall costs. Therefore, there is a need for new products and methods in the current state of the art that can eliminate these challenges.

A review of the literature reveals application numbered TR 2022/009703 as an example of the current state of the art. This application relates to a bispecific chimeric antigen receptor (CAR) polypeptide targeting CD19 in combination with either CD20 or CD22 for the treatment of Acute Lymphoblastic Leukemia (ALL), Hodgkin Lymphoma (HL), or Non-Hodgkin Lymphoma (NHL), as well as the CAR-T cell product developed using this CAR construct. The CAR-T cell described in this application is of allogeneic origin. However, the document does not disclose a minimally manipulated universal CAR-T cell derived from a donor lacking MHC class I molecules.

As a result of the aforementioned limitations and the inadequacy of existing solutions, there is a clear necessity for advancements in the relevant technical field.

### Objective of the Invention

The present invention relates to a method for obtaining a minimally manipulated universal MHC-null CAR-T cell (UN CAR-T CELL) that addresses the above-mentioned requirements, eliminates all existing disadvantages, and offers additional benefits.

The primary objective of the invention is to provide a method for the production of a minimally manipulated universal MHC-null CAR-T cell (UN CAR-T CELL) to be used instead of the genetically manipulated, artificial, whose safety is questionable and potentially tumorigenic cells currently utilized in the field.

The objective of the invention is to enable the use of minimally manipulated universal T cells obtained from donors with a rare group of genetic disorders in which T cell functions are preserved despite the absence of MHC Class I (HLA-A, -B, -C) molecule expression-specifically, individuals diagnosed with Bare Lymphocyte Syndrome Type 1.

Another objective of the invention is to enable the use of only CD45RO-positive memory T cells for CAR-T cell production by completely depleting CD45RA-positive naïve T cells.In this way, UN-CAR-T cells can be obtained from CD45RO positive memory T cells that lack MHC Class I molecules. The invention aims to produce a cellular product with a higher safety profile compared to the existing universal CAR-T cell platforms.

Another objective of the invention is to ensure the production of CAR-T cells that do not have problems related to T cell quality or exhaustion. The invention eliminates the need for additional production time, thereby aiming to deliver the therapy to the patient within 1 day instead of 20 days.

Another objective of the invention is to provide a product and method with a cost significantly lower than that of classical autologous or allogeneic CAR-T cells.

Another objective of the invention is to preserve genetic stability without applying additional genetic modification to the cells thereby enhancing the safety profile. Thus, the invention aims to generate genetically minimally manipulated universal MHC-null CAR-T cells.

Another purpose of the invention is to provide a method that eliminates genetic engineering steps, thus shortening the production time and significantly reducing the unit cost.

In order to achieve the objectives described above, the invention is a method for obtaining minimally manipulated universal CAR-T cells that lack MHC class I molecules, comprising the following process steps:
i. Selection of donors lacking MHC class I molecules (individuals diagnosed with Bare Lymphocyte Syndrome Type 1)
ii. Obtaining peripheral mononuclear cells (PBMCs) from selected donors via apheresis
iii. Isolation of CD3 positive T cells from cells and depletion of CD45RA-positive naïve T cells to obtain CD45RO-positive memory T cells
   or
   Depletion of CD45RA-positive naïve T cells from the cells to obtain CD45RO-positive memory T cells, followed by isolation of CD3-positive cells from these cells.
iv. Transduction of CD45-RO positive memory T cells with the chimeric antigen receptor (CAR) molecule
v. Expansion of CAR-T cells obtained in the previous step and
vi. Aliquoting the expanded CAR-T cells into cryobags each containing 100 million CAR-T cells and storing them frozen until the day of clinical application.

In order to achieve the objectives described above, the invention provides a minimally manipulated universal CAR-T cell that lacks MHC class I molecules.

The structural and characteristic features of the invention, as well as all of its advantages, will be more clearly understood through the detailed description provided below; therefore, the evaluation should be made with reference to this detailed explanation.

### Detailed Description of the Invention

In this detailed description, the product and method which are the subject of the invention are explained solely for the purpose of better understanding the subject matter, and without imposing any limiting effect.

The invention relates to a method for obtaining Minimally Manipulated Universal CAR-T cells lacking MHC class I molecules (MHC-NULL), derived from an individual diagnosed with Bare Lymphocyte Syndrome Type 1. The method comprises the following process steps;
i. Selection of donors that lack MHC class I molecules (Bare Lymphocyte Syndrome Type 1)
ii. Collection of peripheral mononuclear cells (PBMCs) from the selected donor via apheresis
iii. Isolation of CD3 positive T cells from the collected cells and followed by depletion of CD45RA-positive naïve T cells to obtain CD45RO-positive memory T cells,
   or
   Depletion of CD45RA-positive naïve T cells from the collected cells to obtain CD45RO-positive memory T cells, followed by isolation of CD3-positive cells,
iv. Transduction of CD45-RO positive memory T cells with a chimeric antigen receptor (CAR) molecule
v. Expansion of the CAR-T cells obtained in the previous step and
vi. Aliquoting the expanded CAR-T cells into cryobags containing 100 million cells each and cryopreserving them until the day of clinical application.

In step i of the method, the invention involves collecting blood from donors diagnosed with Bare Lymphocyte Syndrome Type 1.

The method of the invention involves collecting samples from donors who lack MHC class I molecules, preferably those diagnosed with Bare Lymphocyte Syndrome Type 1, and who meet the following criteria:
∘ Must be under 40 years old
∘ Must not have T Cell clonality (must not have cancer risk)
∘ Must not have undergone blood and/or organ transplantation
∘ Must not have consumed human flesh
∘ Must not be homosexual
∘ HLA Ab Screening must be Negative
∘ HBV, HCV, HIV, Syphilis must be Negative

In the method of the invention, peripheral blood mononuclear cell apheresis refers to the process of collecting the T lymphocytes required for CAR-T cell production.

In the method of the invention, CD3 selection is performed to isolate the T lymphocytes required for CAR-T Cell production.

In the method of the invention, CD45RA naive T reduction refers to the process of removing naive T lymphocytes that may lead to graft-versus-host disease (GVHD), which are one of the major safety concerns in universal CAR-T cell production due to their potential to cause graft-versus-host disease (GVHD). These CD3 selection and CD45RA reduction steps can be applied flexibly that is, CD45RA depletion may be performed before CD3 selection, or vice versa.

In the method of the invention, the transduction of CD45-RO positive T cells with a CAR (chimeric antigen receptor) includes the transfer of CAR molecule such as CD19, BCMA, GD2, CD22 or newly developed CAR constructs into CD45-RO positive T cells using conventional or novel methods.

In the method of the intervention, the expansion of CD45RO-positive CAR-T cells enables the production of at least 10 billion CAR-T cells in a single manufacturing process.

In the method of the invention, the CAR-T cells produced are cryopreserved in cryobags containing 100 million cells each and stored until the day of intended use.

An embodiment of the method according to the invention is carried out as follows;

A patient who has not been genetically manipulated (modified) and does not naturally express MHC Class I is used as a donor to obtain a sample (sample, blood). This condition is known as Bare Lymphocyte Syndrome Type 1, and the donor must be under 40 years of age, must not have T Cell clonality, must not have undergone blood and/or organ transplantation , must not have consumed human flesh, must not be homosexual, must test negative in HLA antibody screening, and must test negative for HBV, HCV, HIV, and syphilis.

A peripheral blood mononuclear cell (PBMC) sample is obtained from a healthy donor with appropriate qualifications via peripheral blood collection or apheresis.From this mononuclear cell suspension, CD45RA positive naïve T cells, which carry a high risk of alloreactivity, are depleted to isolate CD45RO positive memory T cells. Following CD45RA depletion, the isolation of CD3 positive T cells is an important step for UN CAR-T cell production. The last two steps can be applied in a flexible manner; that is, the process may also follow a sequence in which CD3 positive T cells are first isolated, followed by the depletion of CD45RA positive naïve T cells, thereby resulting in the selection of CD45RO positive T cells.

The production of minimally manipulated universal CAR-T Cell is achieved by transferring the CAR gene using the obtained viral vector, transposon system, sleeping-beauty, electroporation, mRNA, or other methods to be developed.

With the method of the invention, the Minimally manipulated universal CAR-T Cells lacking MHC Class I molecules are subjected to quality control testing by randomly selecting 3 out of every 100 cryobags.

The invention relates to a minimally manipulated universal CAR-T cell (UN CAR-T CELL) lacking MHC class I molecules.

The differences and advantages of the UN CAR-T CELL, which is the subject of the invention, compared to the currently used Autologous and Allogeneic CAR-T Cell are as follows:
1. There is no T Cell quality problem.
2. There is no T Cell fatigue problem.
3. Since no additional time is required for production, it can be delivered to the patient in 1 day instead of 20 days.
4. Its cost is significantly lower than that of classical autologous or allogeneic CAR-T Cell.

## Claims

1. A method for obtaining minimally manipulated universal CAR-T cells lacking MHC class I molecules, ***characterized by comprising;*** the following process steps:
i. Selection of donors lacking MHC class I molecules (Bare Lymphocyte Syndrome Type 1)
ii. Collection of peripheral mononuclear cells (PBMCs) from selected donors via apheresis.
iii. Isolation of CD3 positive T cells from collected cells followed by depletion of CD45RA-positive naïve T cells to obtain CD45RO-positive memory T cells
or
Depletion of CD45RA-positive naïve T cells to obtain CD45RO-positive memory T cells, followed by isolation of CD3-positive T cells from these cells,
iv. Transduction of CD45-RO positive memory T cells with chimeric antigen receptor (CAR) molecule,
v. Expansion of the CAR-T cells obtained in the previous step and
vi. Aliquoting the expanded CAR-T cells into cryobags each containing 100 million CAR-T cells and cryopreserving them until the day of clinical application.

2. The method according to claim *1, **characterizing by comprising;*** in i) process step collecting samples from donors with diagnosis of Bare Lymphocyte Syndrome Type 1 who meet the following criteria
o Must be under 40 years old
o Must not have T Cell clonality
o Must not have undergone blood and/or organ transplantation
o Must not have consumed human flesh
o Must not be homosexual
o Must test negative in HLA Ab Screening
o Must test negative for HBV, HCV, HIV, Syphilis.

3. A minimally manipulated universal CAR-T cell lacking MHC class I molecules obtained by the method according to any of the preceding claims.
